# EUROPEAN PATENT APPLICATION

(11) **EP 4 734 665 A1**
(43) Date of publication of application: **29.04.2026**
(21) Application number: 23958382.6
(22) Date of filing: 08.11.2023
(51) Int. Cl.: H05B 47/105, F24F 8/22, F24F 11/88, A61L 9/20, F24F 120/10

(54) **LAMP DRIVING SYSTEM AND AIR CONDITIONER INCLUDING SAME**

(71) Applicant: LG Electronics Inc., Yeongdeungpo-gu Seoul 07336 (KR)
(72) Inventor: KWAK, Jongwoon, Seoul 06772 (KR)
(74) Representative: Vossius & Partner Patentanwälte Rechtsanwälte mbB
(86) International application number: PCT/KR2023/017809
(87) International publication number: WO 2025/100581

(57) **Abstract**

A lamp driving system and an air conditioner including the same include a lamp, and a lamp driver configured to supply power to the lamp to turn on the lamp, wherein the lamp driver includes a switch, a transformer to which the switch is connected on a primary side and the lamp is connected on a secondary side, and a controller configured to supply a control signal to the switch, wherein the controller performs control such that a first switching frequency for the switch in a first turn-on mode for turning on the lamp is lower than a second switching frequency of a second turn-on mode after the first turn-on mode, and on-duty values of the first turn-on mode and the second turn-on mode are equal.

## Description

### [Technical Field]

The present disclosure relates to a lamp driving system and an air conditioner including the same, and more specifically, to a lamp driving system of a lamp that outputs light for sterilization and an air conditioner including the same.

### [Background Art]

Various air conditioners are being developed to create a pleasant indoor environment. Air conditioners are installed to provide a more pleasant indoor environment to humans by discharging hot and cold air into indoor spaces to control the indoor temperature and purify the indoor air. In general, an air conditioner includes an indoor unit installed indoors and composed of a heat exchanger, and an outdoor unit that is composed of a compressor and a heat exchanger and supplies refrigerant to the indoor unit. In addition, an air conditioner may have at least one indoor unit connected to an outdoor unit, and depending on a requested operating state, supplies refrigerant to the indoor unit and operates in a cooling or heating mode.

An air purifier is a device that purifies polluted air and changes the same into fresh air. An air purifier sucks in polluted air using a fan, collects fine dust or bacteria through a filter, and removes unpleasant odors such as body odor or cigarette odor.

Recently, research is being conducted on technology for sterilizing a certain space or object using light to provide a more hygienic environment.

For example, prior document 1 (Korean Patent Publication No. 1020070094026) relates to an air conditioning device in which a discharge unit 30 and a heat exchanger 20 are disposed in an air passage, and the discharge unit 30 improves the decomposition efficiency of a target component by using streamer discharge, which is a type of plasma discharge.

Prior document 2 (Korean Patent Publication No. 1020190101750) discloses a sterilization module that performs sterilization using ultraviolet and a home appliance equipped with the same.

### [Disclosure]

### [Technical Problem]

An object of the present disclosure is to provide a lamp driving system having a sterilization function using light and an air conditioner including the same.

Another object of the present disclosure is to provide a lamp driving system capable of stably outputting light having a specific wavelength and an air conditioner including the same.

Another object of the present disclosure is to provide a lamp driving system capable of compact design and thus improving degree of freedom of design and an air conditioner including the same.

Another object of the present disclosure is to provide a lamp driving system capable of reducing manufacturing costs and an air conditioner including the same.

### [Technical Solution]

To achieve the above or other objects, a lamp driving system and an air conditioner including the same according to one aspect of the present disclosure can stably output light by controlling a switching frequency while maintaining an on-duty value of a switch for driving a lamp.

To achieve the above or other objects, a lamp driving system and an air conditioner including the same according to one aspect of the present disclosure include a lamp, and a lamp driver configured to supply power to the lamp to turn on the lamp, wherein the lamp driver includes a switch, a transformer to which the switch is connected on a primary side and the lamp is connected on a secondary side, and a controller configured to supply a control signal to the switch, wherein the controller performs control such that a first switching frequency for the switch in a first turn-on mode for turning on the lamp is lower than a second switching frequency of a second turn-on mode after the first turn-on mode, and on-duty values of the first turn-on mode and the second turn-on mode are equal, and the third switching frequency is higher than the second switching frequency.

The controller may perform control with a third switching frequency causing a voltage level lower than a voltage level caused by the second switching frequency in a period in the second turn-on mode.

The period in which the low voltage level is formed may be a non-emitting period in which the lamp does not emit light, and an absolute voltage value in the non-emitting period may be less than an absolute voltage value at which discharge of the lamp is turned off.

The controller may increase the switching frequency from the second switching frequency to a higher fourth switching frequency in a period in the second turn-on mode.

The controller may return the switching frequency from the fourth switching frequency to the second switching frequency.

The controller may decrease the switching frequency from the second switching frequency to a lower fifth switching frequency in a period in the second turn-on mode, and then return the switching frequency to the second switching frequency after a predetermined period of time.

The lamp driving system and the air conditioner including the same according to one aspect of the present disclosure may further include an occupancy detection sensor, wherein the on-duty values of the first turn-on mode and the second turn-on mode may be controlled to be a first duty value if no occupant is detected, and the on-duty values of the first turn-on mode and the second turn-on mode may be controlled to be a second duty value higher than the first duty value if an occupant is detected.

The controller may decrease the switching frequency of the second turn-on mode in a high-sterilization mode.

The on-duty values of the first turn-on mode and the second turn-on mode may be 50% or less.

The on-duty values of the first turn-on mode and the second turn-on mode may be 15% to 50%.

The on-duty values of the first turn-on mode and the second turn-on mode may be 40% to 50%.

The controller may control the switching frequency to linearly increase from the first switching frequency to the second switching frequency.

The controller may control the switching frequency to increase from the first switching frequency to the second switching frequency without having a transient period.

The controller may control the switching frequency to stepwise increase from the first switching frequency to the second switching frequency.

The controller may be connected to a gate terminal of the switch.

The controller may include a gate drive circuit configured to transmit a control signal to the switch, and a frequency conversion circuit connected to an input terminal of the gate drive circuit.

The gate drive circuit may be a general gate driver IC.

The transformer may include a first transformer connected to a first electrode of the lamp and a second transformer connected to a second electrode of the lamp.

The switch may include a first switch connected to the first transformer and a second switch connected to the second transformer, and an inverting terminal may be disposed before the first switch or the second switch.

### [Advantageous effects]

According to at least one embodiment of the present disclosure, it is possible to provide a lamp driving system having a sterilizing function using light and an air conditioner including the same.

According to at least one embodiment of the present disclosure, it is possible to stably output light with a specific wavelength.

According to at least one embodiment of the present disclosure, it is possible to achieve a compact design of a lamp driving system and improve the degree of freedom of design of the lamp driving system and the air conditioner.

According to at least one embodiment of the present disclosure, it is possible to reduce the manufacturing cost of the lamp driving system and the air conditioner including the same.

Meanwhile, various other effects will be directly or implicitly disclosed in the detailed description according to embodiments of the present disclosure which will be described later.

### [Description of Drawings]

FIG. 1 is a block diagram of a main configuration of a lamp driving system according to an embodiment of the present disclosure.
FIG. 2 is a diagram referred to in description of a lamp driving waveform according to an embodiment of the present disclosure.
FIG. 3 is a graph showing changes in power consumption according to a lamp on-duty ratio.
FIG. 4 is a graph showing changes in sterilizing power according to a lamp on-duty ratio.
FIG. 5 is a diagram referred to in description of a lamp driving waveform according to an embodiment of the present disclosure.
FIG. 6 is a diagram referred to in description of a lamp driving waveform according to an embodiment of the present disclosure.
FIG. 7 is a diagram referred to in description of voltage waveforms input to a lamp according to an embodiment of the present disclosure.
FIG. 8 is a diagram referred to in description of a lamp driving waveform according to an embodiment of the present disclosure.
FIG. 9 is a diagram illustrating a circuit configuration of the lamp driving system according to an embodiment of the present disclosure.
FIG. 10 illustrates waveforms at main points of the circuit of FIG. 9.
FIG. 11 illustrates a circuit configuration of a lamp driving system according to an embodiment of the present disclosure.
FIG. 12 illustrates waveforms at main points of the circuit of FIG. 11.
FIG. 13 is a block diagram of a main configuration of an air conditioner according to an embodiment of the present disclosure.

### [Mode for Invention]

Hereinafter, embodiments of the present disclosure will be described in detail with reference to the attached drawings. However, the present disclosure is not limited to these embodiments and may be modified in various forms.

In the drawings, parts that are not related to the description are omitted in order to clearly and briefly describe the present disclosure, and the same reference numerals are used for identical or very similar parts throughout the specification.

Meanwhile, the suffixes "module" and "part" for components used in the following description are given simply for the convenience of writing this specification, and do not have any particularly important meaning or role. Therefore, "module" and "part" may be used interchangeably.

In addition, in this specification, terms such as "first" and "second" may be used to describe various elements, but these elements are not limited by these terms. These terms are used only to distinguish one element from another.

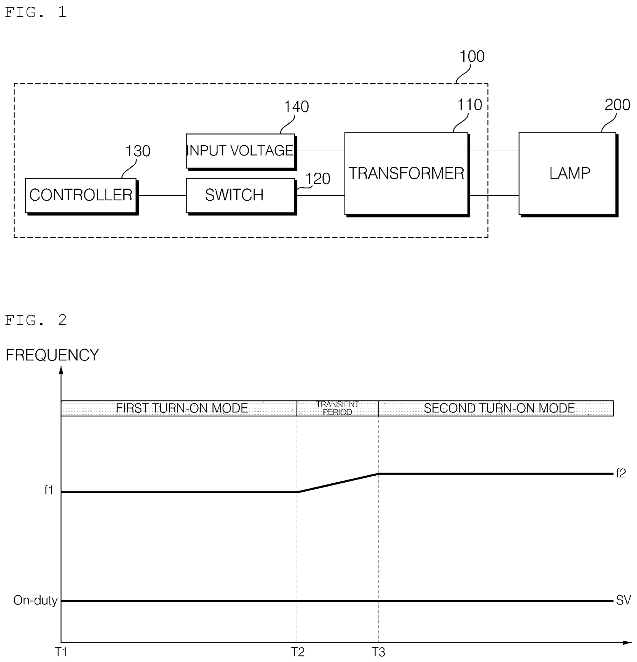

FIG. 1 is a block diagram of a main configuration of a lamp driving system according to an embodiment of the present disclosure.

Referring to FIG. 1, the lamp driving system according to an embodiment of the present disclosure includes a lamp 200 and a lamp driver 100.

The lamp driver 100 supplies power to the lamp 200 to turn on the lamp 200. Electrodes (electrode 1 and electrode 2) of the lamp 200 are connected to the lamp driver 200. The output voltage of the lamp driver 200 is applied to the lamp 200 through the electrodes (electrode 1 and electrode 2).

The lamp 200 is a device that receives a high voltage from the lamp driver 100 and outputs light having a sterilizing effect. For example, the lamp 200 may be an excimer discharge lamp that outputs light with a specific wavelength band. In addition, the lamp 200 may be a mercury discharge lamp, a halogen lamp, a hot cathode discharge lamp, a cold cathode discharge lamp, and other corresponding lamps.

The lamp 200 can sterilize a predetermined space or object by outputting ultraviolet light. Typically, ultraviolet light may be subdivided according to wavelength. For example, ultraviolet light may be classified into UVA with a wavelength of 320 to 400 nm, UVB with a wavelength of 280 to 320 nm, and UVC with a wavelength of 200 to 280 nm. In particular, UVC, which is short-wavelength ultraviolet light, has the characteristics of destroying the DNA of bacteria and causing a chemical reaction in special substances, and thus can be effectively used for sterilization, removal of surface or water contamination, curing, etc.

More preferably, the lamp 200 may be a lamp that produces a wavelength in the far-UVC band (e.g., 200 to 230 nm) in which lamp light is recognized as harmless to the human body. The lamp 200 may be a lamp having a gas composition that generates a wavelength of 222 nm, for example. Alternatively, the lamp 200 may be a lamp that generates a wavelength other than 222 nm (e.g., a wavelength of 207 nm or a wavelength of 234 nm) by changing the gas composition.

Additionally, since the wavelength range that is harmless to the human body is known to be 180 nm to 230 nm, it may also be possible to use ultraviolet light in the 180 nm to 230 nm band. At wavelengths below 180 nm, ozone generation increases in the space when the lamp emits light, and wavelengths above 230 nm may be dangerous to the skin or eyes of the human body. Representative wavelengths in the harmless range for the human body are 207 nm (Kr+Br) from krypton and bromine, and 222 nm (Kr+Cl) from krypton and chloride.

The lamp 200 can sterilize a predetermined space or object by radiating light to the outside according to the control of the lamp driver 100. The lamp driver 100 can turn the lamp 200 on/off by outputting a predetermined signal.

The lamp driver 100 includes a switch 120, a transformer unit 110 including one or more transformers 110 (110a and 110b), and a controller 130 that supplies a control signal to the switch 120.

The switch 120 is connected to the primary side of the transformer 110 and the lamp 200 is connected to the secondary side thereof. In addition, an input voltage circuit 140 is connected to the transformer 110.

The input voltage circuit 140 applies a voltage to an input terminal of a lamp driving inverter circuit to generate a high voltage in the form of a pulse that can cause lamp discharge. For example, the input voltage circuit 140 may be configured as a circuit that applies DC 12 V, or as a circuit that applies DC 24 V or other levels of DC voltage. The circuit configuration of the input voltage circuit 140 may be changed according to each voltage level. The DC power source of the input voltage circuit 140 may be designed as a switched-mode power supply (SMPS) according to the allowable rated capacity of the driving circuit, or a secondary battery such as a battery may be used as the input power source. The input voltage circuit 140 may receive a commercial AC power (e.g., 220 V) in addition to DC power, and additionally include an AC-to-DC power conversion circuit (e.g., a circuit for converting AC 220 V to DC 12 V).

The transformer 110 is a component in which the secondary output voltage and current are determined by the ratio of the number of turns of the winding wound around the core with respect to the primary input voltage and input current. The output characteristics of the transformer 110 may be determined by the coupling force (coupling coefficient) created by the magnetic force of the core.

According to one embodiment of the present disclosure, the lamp driver 100 may be implemented as a circuit having a flyback topology. In this configuration, the switch 120 is connected to the primary side of the transformer 110, and the secondary side of the transformer 110 is directly connected to the electrodes (electrode 1 and electrode 2) such that a voltage is applied to the lamp 200, which is a load.

When the switch 120 is turned on (on-duty), energy is stored in the inductor on the primary side of the transformer 110, and when the switch 120 is turned off (off-duty), the energy stored in the primary side is transferred to the inductor on the secondary side. At this time, the secondary output voltage is formed by the primary/secondary coil turn ratio. The waveform of the output voltage applied to the lamp 200 has a peak-like high-voltage pulse form during a very short period of time (within several microseconds) during an off-duty period.

A high-voltage pulse to be applied to the lamp 200 is generated through the periodic on/off operation of the switch 120. The switch 120 may be a MOSFET, a power transistor, or a high voltage switching element that can be used for switching.

The controller 130 is connected to a gate terminal of the switch 120. The controller 130 can turn the lamp 200 on or off by controlling the operation of the switch 120. For example, the controller 130 may control the frequency and duty ratio of a control signal of the switch 120 using pulse width modulation (PWM) and adjust a signal (voltage) applied to the lamp 200 through the periodic on/off operation of the switch 120.

The controller 130 performs control such that a first switching frequency for the switch 120 in a first turn-on mode (starting mode) in which the lamp 200 is turned on is lower than a second switching frequency in a second turn-on mode (maintenance mode) after the first turn-on mode.

In addition, the controller 130 controls on-duty values of the first turn-on mode (starting mode) and the second turn-on mode (maintenance mode) to be the same.

That is, the controller 130 changes only the frequency of the control signal for the switch 120 while keeping the on-duty value of the control signal constant. Since the controller 130 controls only the frequency, control logic for the lamp 200 can be simplified.

When a sterilizing lamp discharges, ozone is generated in the air. Ozone is generated during plasma discharge and dielectric barrier discharge, and ozone is a gas harmful to the human body and thus it is necessary to minimize the amount of ozone generated during sterilizing discharge.

At the time of using logic that increases the on-duty values when changing from the starting (initiation) mode to the maintenance (stable) mode during discharge of the sterilizing lamp, the amount of ozone generated increases depending on the usage environment and usage time. For example, when the turn-on duty of the switching element is reduced during a short timing in which the discharge mode is changed, the switching element needs to be turned on for only a short time and immediately turned off, which increases the possibility of circuit failure, and accordingly, when the on-duty value is reduced upon mode change, the possibility of ozone generation may increase. Therefore, by fixing the on-duty value without increasing the same when changing the first and second turn-on modes, the increase in the amount of ozone generated can be curbed.

FIG. 2 is a diagram referred to in description of a lamp driving waveform according to an embodiment of the present disclosure.

Referring to FIG. 2, a first time T1 is a time when power is applied (time when the lamp is initially operated). A second time T2 is a time when driving frequency conversion starts. A third time T3 is a time when driving frequency conversion is completed.

A first turn-on mode period between the first time T1 and the second time T2 is a starting frequency period in which lamp discharge smoothly starts at a high voltage level of a low frequency.

A second turn-on mode period after the third time T3 is a stabilization period in which the lamp 200 is turned on and operated with relatively low power. In the second turn-on mode period, the lamp 200 is maintained in an on state at a low voltage level of a high frequency.

The second turn-on mode period is maintained until power is off and is considerably longer than previous periods (the first turn-on mode period and a transient period). The first turn-on mode period requires higher power than the second turn-on mode period, and thus it is desirable to end the first turn-on mode period as rapidly as possible. The period between the second time T2 and the third time T3 is a frequency conversion transient period, which is a period that passes through after the initial first turn-on mode period to move to the second turn-on mode period. Since the transient period needs to minimized to enable stable operation, the transient period can be set as short as possible.

A first switching frequency F1 of the first turn-on mode period is set to a lower value than a second switching frequency F2 of the second turn-on mode period. A switching frequency is the frequency of a control signal of the controller 130 for the switch 120 and can correspond to the frequency of a voltage signal supplied to the lamp 200. The flyback method is a method of collecting energy when the switch 120 is turned on and transferring energy when the switch 120 is turned off. If the switching frequency is low, the duration of one period in which energy is stored increases, and thus the voltage applied to the lamp 200 increases. In the first turn-on mode period, the voltage supplied to the lamp 200 can be increased using the low first switching frequency F1 in order to stably turn on the lamp 200.

An on-duty value SV in the first and second turn-on mode periods is set to be maintained at a constant value. According to one embodiment of the present disclosure, the on-duty value SV in the first and second turn-on modes has a value of 50% or less, and the on-duty value is fixed to the same value in each mode. Therefore, if the on-duty value SV in the first and second turn-on mode periods is set to 50% or less, a general gate driver IC can be used as is. By simply configuring the circuit using the general gate driver IC, the circuit can be configured in a compact size. Accordingly, the general gate driver IC can be used, and complex components that occupy a large PCB area can be eliminated. By reducing the PCB size, the product size can also be reduced and the circuit configuration material cost can be lowered.

Meanwhile, when the on-duty is increased, the amount of ozone generated increases. Even when the second turn-on mode period is maintained, the on-duty value can be maintained constant without increasing compared to the starting turn-on mode period, thereby reducing the amount of ozone generated. Since the second turn-on mode period is relatively much longer than the first turn-on mode period, most ozone generation can occur during maintenance of turn-on. Therefore, it is advantageous in terms of environmental and user protection that the on-duty value in the second turn-on mode period is not large.

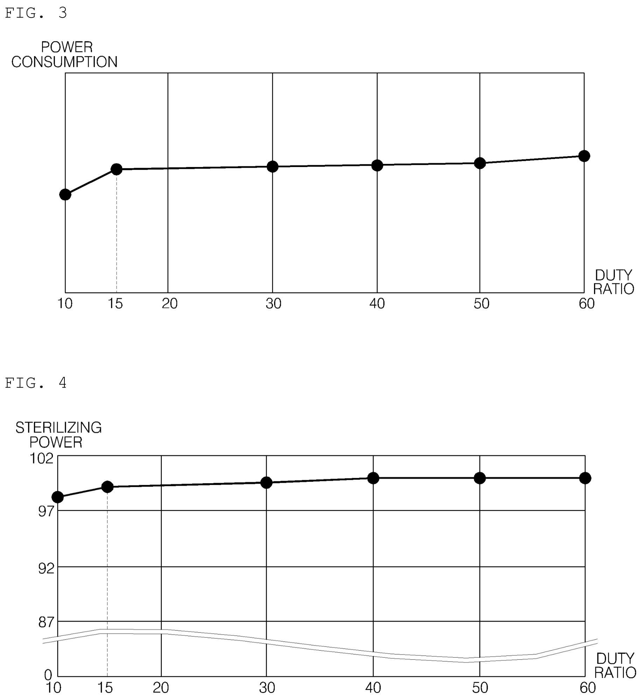

FIG. 3 is a graph showing changes in power consumption according to the lamp on-duty ratio, and FIG. 4 is a graph showing changes in sterilization power according to the lamp on-duty ratio.

The on-duty value SV in the first and second turn-on mode periods may be set to a value of 50% or less. If the on-duty of 50% or less is applied, the gate drive circuit can be configured in various ways, which can improve the degree of freedom of circuit design.

The gate drive circuit may be configured discretely using operational amplifiers, transistors, resistors, capacitors, etc., or may be configured by combining a microcontroller unit (MCU) and a voltage buffer circuit.

In addition, the gate drive circuit may be implemented using a general gate driver IC. In general, the general gate driver IC has its own on-duty value fixed to 50% or less. By using a general gate driver IC, the lamp driver 100 can become compact, and circuits and components of devices equipped with the lamp driver, such as air conditioners, can be used in common.

When the on-duty value is 50% or less, the turn-on time of the switch element decreases, and thus heat generation can be reduced. When heat generation is reduced, parasitic components on the discharge path of the circuit are reduced, which is also advantageous in terms of power consumption. Referring to FIG. 3, when the on-duty value is 15% or less, the output voltage value is low, and thus the amount of light emitted is small and power consumption is also low. Accordingly, when excluding this, power consumption is almost the same under an on-duty value condition in which a desired output voltage is output, but power consumption increases slightly as the on-duty value increases. Therefore, an on-duty value of 50% or less is slightly more advantageous than an on-duty value of 60% in terms of power consumption.

When the on-duty value SV is fixed to a specific value of 50% or less, the circuit scheme that can be provided to developers when implementing the lamp driver can be diversified, thereby increasing the degree of freedom of circuit design and improving the reliability of the driving circuit. It also conforms to ESG by minimizing emission of harmful gases generated by using a lamp.

Meanwhile, in order to stably secure a range of periods in which an intended rated output voltage can be obtained, it is preferable that the on-duty value SV of the first and second turn-on mode periods be set to a specific value within the range of 15% to 50%. When the on-duty value SV is 15% or more, the lamp 200 can be stably turned on with normal driving characteristics and maintained in a turn-on state.

Referring to FIG. 4, when the on-duty value SV is less than 15%, the intended rated output voltage cannot be obtained, and space sterilization power is also reduced.

More preferably, the on-duty value SV of the first and second turn-on mode periods may have a value of 40% to 50%. Referring to FIG. 4, the range of 40% to 50% is a range in which the intended rated output voltage can be obtained and the space sterilization power is the highest within the usable range of 15% to 40%.

If the sterilization power is 99.9%, most germs or bacteria in the space are sterilized, and thus the high sterilization power standard can be considered as 99.9%. Referring to FIG. 4, when the on-duty value is 40% or higher, sterilization power is 99.9%, which can be regarded as high sterilization power.

In addition, from the perspective of driving circuit design, a design that balances the on/off timing of the switching element such that on-time does not exceed 50% is the most ideal. Therefore, the period of 40% to 50% is the most stable operating range in terms of driving stability (reliability).

The controller 130 may control the switching frequency in the transient period such that it linearly increases from the first switching frequency f1 to the second switching frequency f2. Accordingly, abrupt changes can be prevented.

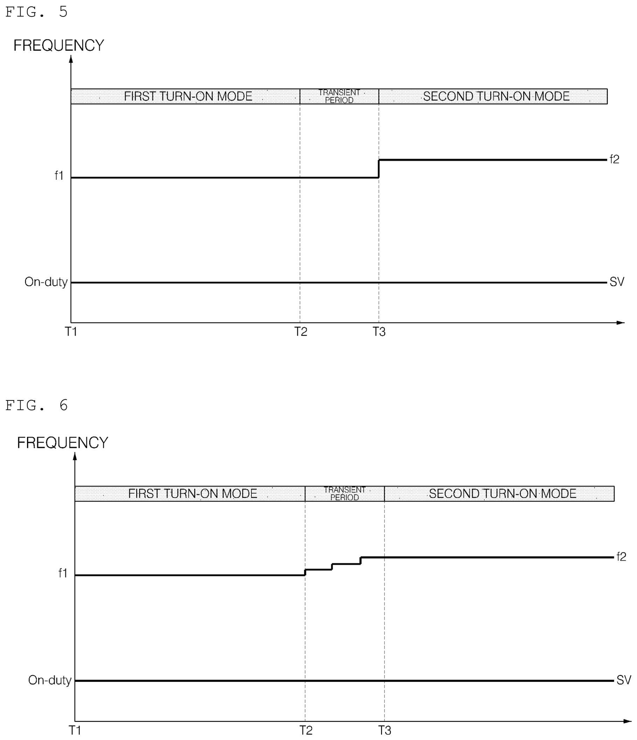

FIG. 5 is a diagram referenced to in description of a lamp driving waveform according to an embodiment of the present disclosure.

Referring to FIG. 5, the controller 130 may control the switching frequency such that it increases from the first switching frequency f1 to the second switching frequency f2 without a transient period in which the switching frequency increases linearly.

The difference between the present embodiment and the embodiment of FIG. 4 is that the switching frequency jumps from the first switching frequency f1 to the second switching frequency f2 without a linear increase. Accordingly, the transient period can be minimized.

FIG. 6 is a diagram referred to in description of the lamp driving waveform according to an embodiment of the present disclosure.

Referring to FIG. 6, the controller 130 may control the switching frequency such that it increases in a stepwise manner. The controller 130 may control the switching frequency such that it increases in a stepwise manner from the first switching frequency f1 to the second switching frequency f2 in the transient period.

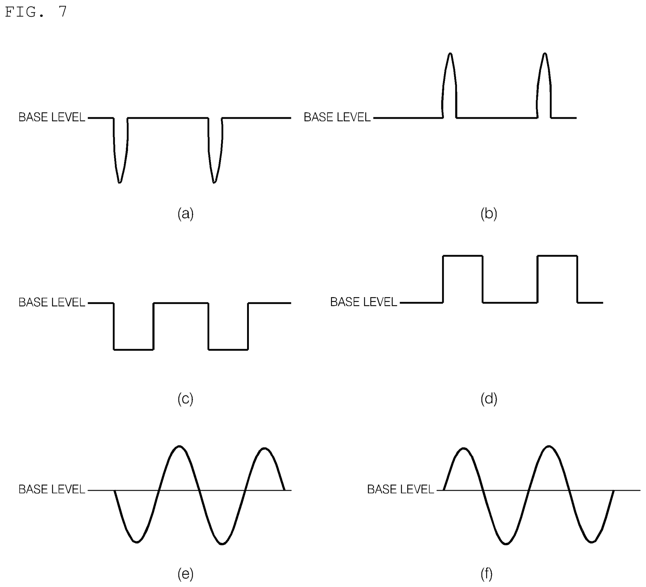

FIG. 7 is a diagram referenced to in description of voltage waveforms input to the lamp according to the embodiment of the present disclosure. (a) and (b) of FIG. 7 illustrate examples of output waveforms possible in a flyback type circuit structure, (c) and (d) of FIG. 7 illustrate examples of output waveforms possible in a half bridge type circuit structure, and (e) and (f) of FIG. 7 illustrate examples of output waveforms possible in a sine wave generating circuit structure.

Since an AC voltage can turn on a high-voltage lamp, the lamp driver 200 that receives a DC voltage inputs the final output voltage waveform in an AC form to the lamp 200. There are various AC voltage forms.

This specification mainly describes a flyback type inverter structure, and referring to (a) and (b) of FIG. 7, a pulse waveform is maintained only for a short time of several microseconds (us) during a certain period and returns to a base level (ground level) for the remainder of the period in the flyback structure.

The embodiments of the present disclosure are not limited to the flyback structure, as the duty value is fixed in the first and second turn-on modes.

Therefore, as in shown in (c) and (d) of FIG. 7, the embodiments of the present disclosure can also be applied to a circuit structure in which the output waveform of the lamp driver 200 is in the form of a sine wave (sine-wave pulse).

In addition, as shown in (e) and (f) of FIG. 7, the embodiments of the present disclosure can also be applied to a circuit structure in which an output in the form of a square-wave pulse is generated.

In addition, the embodiments of the present disclosure are applicable to structures in which the output waveform is a positive pulse and a negative pulse.

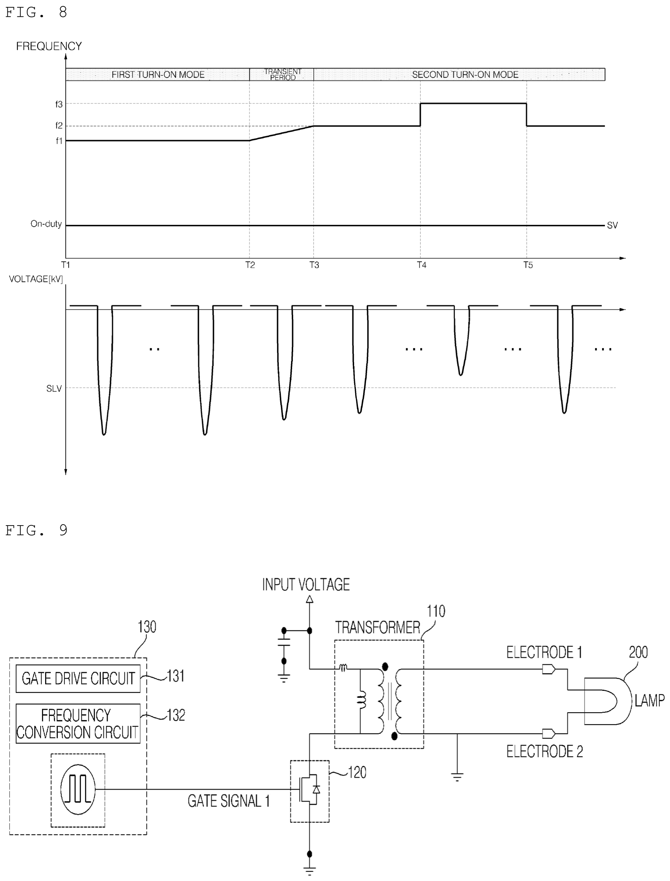

FIG. 8 is a diagram referred to in description of a lamp driving waveform according to an embodiment of the present disclosure.

Referring to FIG. 8, the second turn-on mode may include a period T3 to T4 in which first control is performed to repeat on/off at a specific frequency while maintaining a turn-on state, and a period T4 to T5 in which second control is performed to repeat on/off at a high frequency causing a low voltage level that does not cause discharge (does not emit light) in the lamp 200.

The controller 130 may perform control using a third switching frequency f3 that causes a lower voltage level than the voltage level according to the second switching frequency f2 in some periods of the second turn-on mode period. And the third switching frequency is higher than the second switching frequency. Here, the period T4 to T5 in which a low voltage level is formed may be a non-emitting period in which the lamp 200 does not emit light.

That is, the present embodiment differs from the embodiment described with reference to FIG. 2 in that the non-emitting period T4 to T5 in which the lamp 200 does not emit light is present within the second turn-on mode period.

The absolute voltage value in the non-emitting period T4 to T5 is less than the absolute voltage value SLV at which discharge of the lamp 200 is turned off. Accordingly, a sufficient high voltage to maintain the turn-on state is not applied to the lamp 200, and thus light is not emitted.

Meanwhile, the non-emitting period T4 to T5 may be relatively longer than a previous first control period T3 to T4.

In general, the lamp 200 is turned off to stop light emission. If the off state is prolonged, more energy is required when the lamp 200 returns to the first turn-on mode for re-emission due to the characteristics of the excimer lamp, and discharge may not occur smoothly.

According to one embodiment of the present disclosure, since a small amount of energy is continuously supplied to the lamp 200 in the non-emitting period T4 to T5, it is possible to re-enter a lamp maintenance emission mode (after T5) by returning to the first control of the second turn-on mode without having to return to the first turn-on mode for re-emission, and at this time, the excimer lamp easily emits light.

In addition, the present embodiment can be applied in an area where a light amount restriction regulation is applied. The non-emitting period T4 to T5 may be a timing period for the purpose of turning off lamp discharge due to light amount restriction, etc.

According to an embodiment, the controller 130 may increase the switching frequency for the switch 120 from the second switching frequency f2 to a fourth switching frequency (not shown) that is higher during some periods in the second turn-on mode (maintenance mode). The controller 130 may decrease the output voltage level and reduce the amount of light by increasing the frequency.

Thereafter, the controller 130 may restore the switching frequency from the fourth switching frequency to the second switching frequency f2 to restore the normal second turn-on mode.

If it is desired to limit the amount of light during the second turn-on mode, the controller 130 may further increase the frequency after a certain period of time from the second turn-on mode operation (while maintaining the on-duty ratio) to decrease the output voltage level, thereby reducing the amount of light. In addition, in order to increase the decreased amount of light to the previous state, the controller 130 may restore the frequency to the second turn-on mode.

On the other hand, an embodiment of decreasing the frequency to increase the amount of light in the normal second turn-on mode is also possible. The controller 130 may return the switching frequency from the second switching frequency f2 to a fifth switching frequency that is lower during some periods in the second turn-on mode (maintenance mode) .

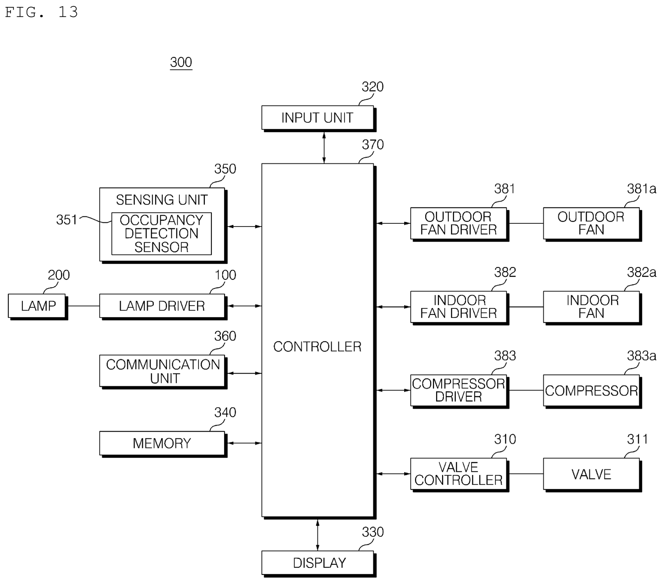

The lamp driving system according to an embodiment of the present disclosure may further include an occupancy detection sensor (not shown). Alternatively, the lamp driver 200 may receive sensing data from an occupancy detection sensor (refer to 351 in FIG. 13) of a home appliance such as an air conditioner or may receive a control signal from a controller (refer to 370 in FIG. 13) of the home appliance.

If an occupant is not detected, the on-duty value of the first turn-on mode (starting mode) and the second turn-on mode (maintenance mode) is controlled to a first duty value.

If an occupant is detected by the occupancy detection sensor, the controller 130 may increase the on-duty value of the first turn-on mode (starting mode) and the second turn-on mode (maintenance mode) to a second duty value (not shown) greater than the first duty value SV. As the duty value increases, the amount of light can be reduced.

According to one embodiment of the present disclosure, the on-duty value can be changed (decreased or increased) in a specific mode. For example, if a user wants to arbitrarily change the amount of light when a constant amount of light is emitted in the normal mode, the desired purpose can be achieved by changing the on-duty value. When the amount of light is to be decreased, the desired purpose can be achieved by setting the on-duty to a value outside a light amount stabilization range without changing the frequency. For example, when the light amount stabilization on-duty range is 15 to 50%, a small amount of lamp light can be achieved by setting the on-duty value to 10*.

According to one embodiment of the present disclosure, the controller 130 may improve the sterilizing power by lowering the switching frequency of the second turn-on mode (maintenance mode) and increasing the voltage applied to the lamp 200.

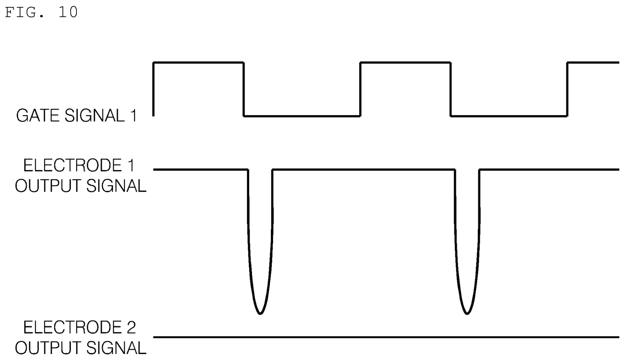

FIG. 9 illustrates a circuit configuration of the lamp driving system according to an embodiment of the present disclosure and shows an example of the lamp driving system of FIG. 1. FIG. 10 illustrates waveforms at main points of the circuit of FIG. 9.

Referring to FIG. 9, the controller 130 may include a gate drive circuit 131 that transmits a control signal to the switch 120, and a frequency conversion circuit 132 connected to an input terminal of the gate drive circuit 131.

The gate drive circuit 131 is a circuit that generates an input signal applied to the gate terminal of the switch 120 such that the switch 120 can be turned on and off. The gate drive circuit 131 may be discretely configured by a combination of components such as an operational amplifier, a transistor, a resistor, and a capacitor, or may be configured using a general gate driver IC.

By configuring the gate drive circuit 131 using a general gate driver IC, the number of parts can be reduced and the circuit area can be decreased to reduce the circuit configuration material cost. In general, circuit reliability is inversely proportional to the number of parts. Therefore, by applying a general IC, the effect of improving circuit reliability can also be obtained. In addition, the gate driver circuit 131 may be formed by combining a microcontroller unit (MCU) and a voltage buffer IC (voltage amplification IC).

The frequency conversion circuit 132 is a circuit block for varying the frequency of the first and second turn-on modes when the sterilizing high-voltage lamp 200 is operated. The frequency conversion circuit 132 may be connected to a pin serving to set a frequency in the gate driver circuit 131. For example, the frequency conversion circuit 132 may be configured using a resistor, a transistor, and one control signal connected to the frequency setting pin of the gate driver circuit 131.

The frequency setting pin of the gate driver circuit 131 sets a desired frequency by an RC time constant value of passive elements of a resistor and a capacitor outside the pin. The frequency may be changed by adding a resistor, a transistor, and a control signal to the frequency setting pin.

Referring to FIG. 9 and FIG. 10, the controller 130 applies a control signal (gate signal 1) having a predetermined frequency and duty ratio to the gate of the switch 120.

According to the control signal (gate signal 1), when the switch 120 is turned on, energy is stored in the inductor on the primary side of the transformer 110, and when the switch 120 is turned off, the energy stored in the primary side is transferred to the inductor on the secondary side. At this time, the output voltage on the secondary side is formed by the primary/secondary coil turn ratio.

When an output signal in the form of a peak-like high-voltage pulse is applied to electrode 1, and the voltage absolute value of the applied output signal is greater than the voltage absolute value that can cause discharge, the lamp 200 can be turned on.

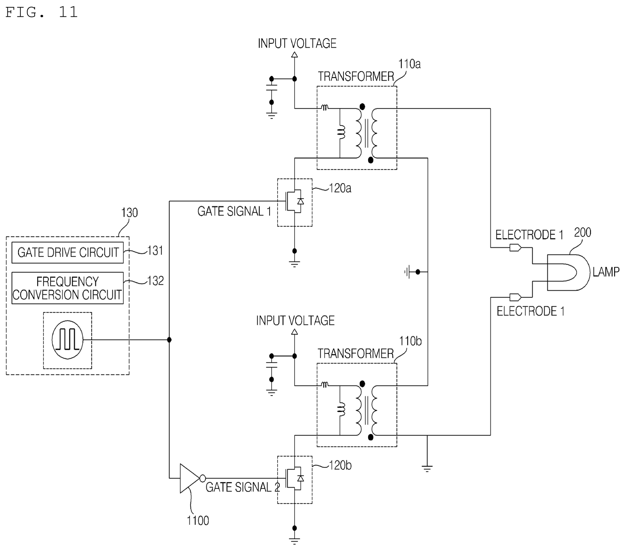

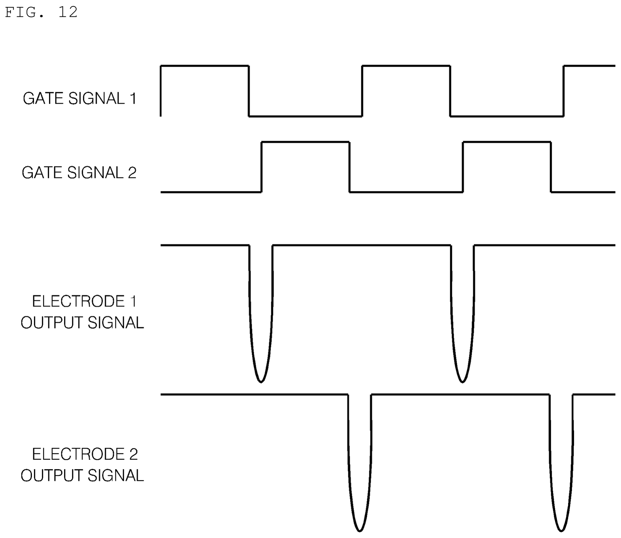

FIG. 11 illustrates a circuit configuration of the lamp driving system according to an embodiment of the present disclosure, and shows an example of the lamp driving system of FIG. 1. FIG. 12 illustrates waveforms at main points of the circuit of FIG. 11.

The embodiment illustrated in FIG. 11 and FIG. 12 differs from the embodiment illustrated in FIG. 9 and FIG. 10 in that the former includes two transformers 110a and 110b and two switches 120a and 120b.

Referring to FIG. 11, the transformer 110 may include a first transformer 110a connected to the first electrode (electrode 1) of the lamp 200 and a second transformer 110b connected to the second electrode (electrode 2) of the lamp 200.

In addition, the switch 120 may include a first switch 120a connected to the primary side of the first transformer 110a and a second switch 120b connected to the second transformer 110b.

An inverting terminal 1100 may be disposed before the first switch 120a or the second switch 120b.

Referring to FIG. 11 and FIG. 12, when gate signal 1 is applied to the first switch 120a, gate signal 2 is applied to the second switch 120b due to the inverting terminal 1100.

According to gate signal 1, when the first switch 120a is turned on, energy is stored in the inductor on the primary side of the first transformer 110a, and when the first switch 120a is turned off, the energy stored in the primary side is transferred to the inductor on the secondary side. At this time, the output voltage on the secondary side is formed by the primary/secondary coil turn ratio of the first transformer 110a.

According to gate signal 2, when the second switch 120b is turned on, energy is stored in the inductor on the primary side of the second transformer 110b, and when the second switch 120b is turned off, the energy stored in the primary side is transferred to the inductor on the secondary side. At this time, the output voltage on the secondary side is formed by the primary/secondary coil turn ratio of the second transformer 110b.

The present embodiment can be used when a larger amount of light is required at the same frequency. Theoretically, a pulse that causes the potential difference between electrode 1 and electrode 2 to be a potential difference that can cause discharge at the same frequency can be applied twice. Therefore, twice the luminosity can be achieved at the same frequency.

FIG. 13 is a block diagram of a main configuration of an air conditioner according to one embodiment of the present disclosure.

Referring to FIG. 13, the air conditioner 300 includes the lamp driving system (the lamp driver 100 and the lamp 200) described with reference to FIG. 1 to FIG. 12. The lamp driver 100 drives the lamp according to control of a controller 370, thereby sterilizing a predetermined area of the space in which the air conditioner 300 is installed.

The lamp 200 may be placed on the front of the indoor unit of the air conditioner 300. The indoor unit of the air conditioner 300 may be installed on the ceiling or wall of an indoor space or may be used by being placed at a specific position as a stand-alone unit. Since the indoor unit is placed such that at least an area thereof faces the indoor space, the lamp 200 can be disposed such that it emits light to the outside from one surface of the indoor unit.

Referring to FIG. 13, the air conditioner 300 may include a compressor 383a, an outdoor fan 381a, an indoor fan 382a, a controller 370, a sensing unit 350, a communication unit 360, and a memory 340. In addition, the air conditioner 300 may further include a compressor driver 383, an outdoor fan driver 381, an indoor fan driver 382, various valves 311 such as a switching valve and an expansion valve, a display 330, and an input unit 320.

The compressor 383a, the outdoor fan 381a, and the indoor fan 382a may operate as described above with reference to FIG. 1 and FIG. 2.

The input unit 320 is provided with a plurality of operation buttons and transmits a signal for a target operating temperature of the air conditioner to the controller 370.

The display 330 may display the operating status of the air conditioner 300. For example, the display 330 may include a display means that outputs the operating status of the indoor unit 20 to display the operating status and errors.

The display 330 may also display the wiring status of the indoor unit 20 and the outdoor unit 30. For example, the display 330 may include a light emitting diode (LED), and the light emitting diode (LED) can be turned on when the wiring status of a communication line and/or a power line is normal and can be turned off when the wiring status of the communication line and/or the power line is abnormal.

The sensing unit 350 may include a plurality of sensors to obtain data related to the operation and status of the air conditioner 300. The sensing unit 350 may include various sensors to obtain cycle operation data.

For example, the sensing unit 350 may include a plurality of temperature sensors. A discharge temperature sensor may detect a refrigerant discharge temperature in the compressor 383a and transmit a signal representing the detected refrigerant discharge temperature to the controller 370. An outdoor temperature sensor may detect the outdoor temperature, which is the temperature around the outdoor unit 30 of the air conditioner 300, and transmit a signal representing the detected outdoor temperature to the controller 370. An indoor temperature sensor may detect the indoor temperature, which is the temperature around the indoor unit 20 of the air conditioner 300, and transmit a signal representing the detected indoor temperature to the controller 370.

The controller 370 may control the air conditioner 300 to operate on the basis of at least one of the detected refrigerant discharge temperature, the detected outdoor temperature, or the detected indoor temperature, and an input target temperature. For example, the controller 370 may control the air conditioner 300 to operate by calculating final target superheat.

In addition, the sensing unit 350 may include a humidity sensor, a pressure sensor, and other sensors that can obtain data related to the operation and status of the air conditioner 300 and transmit sensing data of the sensors to the controller 370.

In addition, the sensing unit 350 further includes an occupancy detection sensor 351. The occupancy detection sensor 351 may detect whether a person is present in the air-conditioned space where the indoor unit is disposed.

For example, the occupancy detection sensor 351 may use a passive infrared (PIR) sensor. The PIR sensor is a sensor that detects an object that emits infrared and is composed of an infrared emitter and a light receiving sensor. When infrared rays emitted from the infrared emitter are reflected from a person, the light receiving sensor can sense the reflected light to detect occupancy.

More preferably, the occupancy detection sensor 351 may use a high-performance sensor of another type, such as a RADAR sensor or an impulse response-ultra wide band (IR-UWB) sensor, to not only detect occupants but also more precisely determine a position of a user.

The occupancy detection sensor 351 may include a camera sensor. The camera sensor can capture images of the air-conditioned space where the indoor unit is placed. The indoor unit can recognize a user, an object, a space, etc. by recognizing images acquired through the camera sensor.

The controller 370 may control the air conditioner 300 on the basis of sensing data obtained by the sensing unit 350.

The controller 370 may control the compressor driver 383, the outdoor fan driver 381, the indoor fan driver 382, and a valve controller 310, as shown in the figure, to control the operations of the compressor 383a, the indoor fan 382a, and the outdoor fan 381a.

For example, the controller 370 may output speed command signals to the compressor driver 383, the outdoor fan driver 381, and the indoor fan driver 382 on the basis of the target temperature.

The compressor motor, the outdoor fan motor, and the indoor fan motor may be operated at target rotation speeds on the basis of the speed command signals.

The controller 370 may control the overall operation of the air conditioner 300 in addition to controlling the compressor driver 383, the outdoor fan driver 381, or the indoor fan driver 382.

For example, the controller 370 may control the operation of a cooling/heating switching valve or a four-way valve through the valve controller 310. Further, the controller 370 may control the operation of an expansion mechanism or an expansion valve 106 through the valve controller 310.

The air conditioner may further include a power supply (not shown) that supplies power to the respective units, such as the compressor 383a, the outdoor fan 381a, the indoor fan 382a, the controller 370, and the memory 340.

The memory 340 may store data necessary for the operation and control of the air conditioner 300. In addition, the memory 340 may store image data acquired through a camera module 355 and sensing data acquired through the sensing unit 350.

The communication unit 360 may be equipped with one or more communication modules to transmit/receive data to/from other devices in a wired or wireless manner.

The controller 370 may transmit status information of the air conditioner 300 to other devices, such as a server (not shown), through the communication unit 360. For example, the communication unit 360 may be controlled such that image data acquired through the camera module 355 and sensing data acquired through the sensing unit 350 are transmitted to other devices such as a server.

In addition, the controller 360 may control the air conditioner 300 on the basis of control signals and various types of data received from other devices such as a server.

Although the preferred embodiments of the present disclosure have been illustrated and described above, the present disclosure is not limited to the specific embodiments described above, and various modifications can be made by those skilled in the art to which the present disclosure pertains without departing from the gist of the present disclosure claimed in the patent claims, and such modifications should not be individually understood from the technical idea or prospect of the present disclosure.

## Claims

1. A lamp driving system comprising:
a lamp; and
a lamp driver configured to supply power to the lamp to turn on the lamp,
wherein the lamp driver comprises:
a switch;
a transformer to which the switch is connected on a primary side and the lamp is connected on a secondary side; and
a controller configured to supply a control signal to the switch,
wherein the controller performs control such that a first switching frequency for the switch in a first turn-on mode for turning on the lamp is lower than a second switching frequency of a second turn-on mode after the first turn-on mode, and on-duty values of the first turn-on mode and the second turn-on mode are equal.

2. The lamp driving system of claim 1, wherein the controller performs control with a third switching frequency causing a voltage level lower than a voltage level caused by the second switching frequency in a period in the second turn-on mode, and the third switching frequency is higher than the second switching frequency.

3. The lamp driving system of claim 2, wherein the period in which the low voltage level is formed is a non-emitting period in which the lamp does not emit light, and an absolute voltage value in the non-emitting period is less than an absolute voltage value at which discharge of the lamp is turned off.

4. The lamp driving system of claim 1, wherein the controller increases the switching frequency from the second switching frequency to a higher fourth switching frequency in a period in the second turn-on mode.

5. The lamp driving system of claim 4, wherein the controller returns the switching frequency from the fourth switching frequency to the second switching frequency.

6. The lamp driving system of claim 1, wherein the controller decreases the switching frequency from the second switching frequency to a lower fifth switching frequency in a period in the second turn-on mode, and then returns the switching frequency to the second switching frequency after a predetermined period of time.

7. The lamp driving system of claim 1, further comprising an occupancy detection sensor,
wherein the on-duty values of the first turn-on mode and the second turn-on mode are controlled to be a first duty value if no occupant is detected, and the on-duty values of the first turn-on mode and the second turn-on mode are controlled to be a second duty value higher than the first duty value if an occupant is detected.

8. The lamp driving system of claim 1, wherein the controller decreases the switching frequency of the second turn-on mode in a high-sterilization mode.

9. The lamp driving system of claim 1, wherein the on-duty values of the first turn-on mode and the second turn-on mode are 50% or less.

10. The lamp driving system of claim 1, wherein the on-duty values of the first turn-on mode and the second turn-on mode are 15% to 50%.

11. The lamp driving system of claim 1, wherein the on-duty values of the first turn-on mode and the second turn-on mode are 40% to 50%.

12. The lamp driving system of claim 1, wherein the controller controls the switching frequency to linearly increase from the first switching frequency to the second switching frequency.

13. The lamp driving system of claim 1, wherein the controller controls the switching frequency to increase from the first switching frequency to the second switching frequency without having a transient period.

14. The lamp driving system of claim 1, wherein the controller controls the switching frequency to stepwise increase from the first switching frequency to the second switching frequency.

15. The lamp driving system of claim 1, wherein the controller is connected to a gate terminal of the switch.

16. The lamp driving system of claim 15, wherein the controller comprises:
a gate drive circuit configured to transmit a control signal to the switch; and
a frequency conversion circuit connected to an input terminal of the gate drive circuit.

17. The lamp driving system of claim 16, wherein the gate drive circuit is a general gate driver IC.

18. The lamp driving system of claim 1, wherein the transformer includes a first transformer connected to a first electrode of the lamp and a second transformer connected to a second electrode of the lamp, the switch includes a first switch connected to the first transformer and a second switch connected to the second transformer, and an inverting terminal is disposed before the first switch or the second switch.

19. An air conditioner comprising:
a lamp; and
a lamp driver configured to supply power to the lamp to turn on the lamp,
wherein the lamp driver comprises:
a switch;
a transformer to which the switch is connected on a primary side and the lamp is connected on a secondary side; and
a controller configured to supply a control signal to the switch,
wherein the controller performs control such that a first switching frequency for the switch in a first turn-on mode for turning on the lamp is lower than a second switching frequency of a second turn-on mode after the first turn-on mode, and on-duty values of the first turn-on mode and the second turn-on mode are equal.

20. The air conditioner of claim 19, further comprising an occupancy detection sensor,
wherein the on-duty values of the first turn-on mode and the second turn-on mode are controlled to be a first duty value if no occupant is detected, and the on-duty values of the first turn-on mode and the second turn-on mode are controlled to be a second duty value higher than the first duty value if an occupant is detected.
